# EUROPÄISCHE PATENTSCHRIFT

(11) **EP 2 717 965 B1**
(45) Veröffentlichungstag und Bekanntmachung des Hinweises auf die Patenterteilung: **01.11.2017**
(21) Anmeldenummer: 12723640.4
(22) Anmeldetag: 18.05.2012
(51) Int. Cl.: A61P 31/10, A61P 31/12, A61P 31/04, A61K 38/16, A61K 31/785

(54) **VERWENDUNG VON EPSILON-POLY-L-LYSIN ZUR ERHÖHUNG DER BIOVERFÜGBARKEIT ANTIMIKROBIELLER PEPTIDE IN MENSCHLICHER HAUT**
USE OF EPSILON-POLY-L-LYSINE FOR INCREASING THE BIOAVAILABILITY OF ANTIMICROBIAL PEPTIDES IN THE HUMAN SKIN
UTILISATION DE EPSILON-POLY-L-LYSINE POUR AUGMENTER LA BIODISPONIBILITÉ DE PEPTIDES ANTIMICROBIENS DANS LA PEAU HUMAINE

(30) Priorität: 07.06.2011 DE 102011077052
(43) Veröffentlichungstag der Anmeldung: 16.04.2014
(73) Patentinhaber: Beiersdorf AG, 20253 Hamburg (DE)
(72) Erfinder: LORENZ, Janina, 22607 Hamburg (DE); THIESEN, Kathrin, 22301 Hamburg (DE); BRAREN, Sandra, 20251 Hamburg (DE); TERSTEGEN, Lara, 20253 Hamburg (DE)
(74) Vertreter: Wilke, Jochen
(86) Internationale Anmeldenummer: PCT/EP2012/059235
(87) Internationale Veröffentlichungsnummer: WO 2012/168050

(56) Entgegenhaltungen:
- JP-A- 2003 171 464
- JP-A- 2004 067 587

## Beschreibung

Gegenstand der Erfindung ist die Verwendung von epsilon-Poly-L-Lysin (εPL) zur Freisetzung antimikrobieller Peptide in menschlicher Haut. In besonderen Ausführungsformen betrifft die vorliegende Erfindung kosmetische und dermatologische Zubereitungen, die εPL enthalten.

Der gesunde warmblütige Organismus, insbesondere auch die gesunde menschliche Haut, ist mit einer Vielzahl nichtpathogener Mikroorganismen besiedelt. Diese sogenannte Mikroflora der Haut ist nicht nur unschädlich, sie stellt einen wichtigen Schutz zur Abwehr opportunistischer oder pathogener Keime dar.

Bakterien gehören zu den prokaryotischen Einzellern. Sie können grob nach ihrer Form (Kugel, Zylinder, gekrümmter Zylinder) sowie nach dem Aufbau ihrer Zellwand (grampositiv, gramnegativ) unterschieden werden. Feinere Unterteilungen tragen auch der Physiologie der Organismen Rechnung. So existieren aerobe, anaerobe sowie fakultativ anaerobe Bakterien. Manche Individuen sind in ihrer Eigenschaft als pathogene Keime von medizinischer Bedeutung, andere wiederum sind vollkommen harmlos.

Gegen Bakterien wirksame Substanzen sind seit geraumer Zeit bekannt. Der Begriff "Antibiotika" beispielsweise, der nicht auf alle antimikrobiell wirksamen Substanzen anwendbar ist, läßt sich auf das Jahr 1941 datieren, obwohl die ersten Erkenntnisse zum Penicillin bereits im Jahre 1929 gefunden wurden. Antibiotika im heutigen Sinne sind nicht für alle medizinischen, schon gar nicht kosmetische Anwendungen geeignet, da häufig auch der warmblütige Organismus, also etwa der erkrankte Patient, bei Anwendung auf irgendeine Weise in seinen Stoffwechselfunktionen beeinträchtigt wird.

Eine Aufgabe der vorliegenden Erfindung war also, den Stand der Technik in dieser Richtung zu bereichern, insbesondere Substanzen zur Verfügung zu stellen, welche gegen grampositive und/oder gramnegative Bakterien wirksam sind, ohne dass mit der Anwendung der Substanzen eine unvertretbare Beeinträchtigung der Gesundheit des Anwenders verbunden wäre.

Gramnegative Keime sind beispielsweise Escherichia coli, Pseudomonas-Arten sowie Enterobacteriaceen, wie etwa Citrobacter freundii.

Auch grampositive Keime spielen in Kosmetik und Dermatologie eine Rolle. Bei der unreinen Haut beispielsweise sind neben anderen Einflüssen bakterielle Sekundärinfektionen von ätiologischer Bedeutung. Einer der wichtigsten Mikroorganismen, der in Zusammenhang mit unreiner Haut steht, ist Propionibacterium acnes.

Unreine Haut und/oder Komedonen beeinträchtigen das Wohlbefinden der Betroffenen auch selbst in leichten Fällen. Da praktisch jeder oder jede Jugendliche von unreiner Haut irgendeiner Ausprägung betroffen ist, besteht bei vielen Personen Bedarf, diesem Zustande abzuhelfen.

Eine besondere Aufgabe der vorliegenden Erfindung war es also, einen gegen unreine Haut bzw. Propionibacterium acnes wirksamen Stoff bzw. Stoffkombination zu finden.

Dermatomycosen sind Krankheiten, bei der gewisse Pilzarten, insbesondere Dermatophyten, in die Haut und Haarfollikel eindringen. Die Symptome von Dermatomycosen sind beispielsweise Bläschen, Exfoliation, Rhagaden und Erosion, meist verbunden mit Juckreiz oder allergischem Ekzem.

Zu den pathogenen und fakultativ pathogenen Keimen gehören beispielsweise aus der Gruppe der Hefen Candida-Arten (z.B. Candida albicans) und solche der Familie Mallassezia. Malassezia-Arten, insbesondere Malassezia furfur, sind für Hauterkrankungen wie Pityriasis versicolor, Seborrhoe in den Formen Seborrhoea oleosa und Seborrhoea sicca, welche sich vor allem als Seborrhoea capitis (= Kopfschuppen) äußern, seborrhoisches Ekzem und Pityrosporum-Follikulitis verantwortlich zu machen. Eine Beteiligung von Malassezia furfur an der Entstehung von Psoriasis wird von der Fachwelt diskutiert.

Alle Bereiche der menschlichen Haut können von Dermatomycosen befallen werden. Dermatophytien befallen fast ausschließlich Haut, Haare und Nägel. Hefemycosen können auch Schleimhäute und innere Organe befallen, Systemmycosen erstrecken sich regelmäßig auf ganze Organsysteme.

Besonders häufig sind die Körperbereiche betroffen, auf welchen sich durch Kleidung, Schmuck oder Schuhwerk Feuchtigkeit und Wärme stauen können. So gehört der Fußpilz zu den bekanntesten und am weitesten verbreiteten Dermatomycosen. Besonders unangenehm sind weiterhin Pilzerkrankungen der Finger- und Fußnägelbereiche.

Ferner sind "Superinfektionen" der Haut durch Pilze und Bakterien nicht selten. Hierbei wird die bestehende physiologische Keimbesiedelung der Haut, durch Neuinfektion mit hohen Keimzahlen eines oder mehrerer anderer Erreger, beispielsweise Staphylokokken oder Candida albicans, überwuchert. Dies kann insbesondere bei Zusammentreffen ungünstiger äußerer Einflüsse oder Schwächung der Abwehrlage des Körpers auftreten.

Solche Superinfektionen können sich, in Abhängigkeit vom betreffenden Keim, in günstig verlaufenden Fällen in unangenehmen Hauterscheinungen (Juckreiz, unschönes äußeres Erscheinungsbild) äußern. In ungünstig verlaufenden Fällen können sie aber zu großflächiger Zerstörung der Haut führen, im schlimmsten Falle sogar im Tode des Patienten gipfeln.

Derartige Superinfektionen werden bei einer Vielzahl dermatologischer Erkrankungen, z.B. atopischem Ekzem, Neurodermitis, Akne, seborrhoischer Dermatitis oder Psoriasis beobachtet. Auch viele medizinische und therapeutische Maßnahmen, z.B die Radio- oder Chemotherapie von Tumorerkrankungen, als Nebenwirkung hervorgerufene, medikamentös induzierte Immunsuppression oder aber systemische Antibiotikabehandlung, ebenso wie externe chemische oder physikalische Einflüsse (z.B. Umweltverschmutzung, Smog), fördern das Auftreten von Superinfektionen der äußeren und inneren Organe, insbesondere der Haut und der Schleimhäute.

Zwar ist es im Einzelfalle ohne weiteres möglich, Superinfektionen mit Antibiotika zu bekämpfen, meistens haben solche Substanzen aber den Nachteil unangenehmer Nebenwirkungen. Oft sind Patienten beispielsweise gegen Penicilline allergisch, weswegen eine entsprechende Behandlung sich in einem solchen Falle verbieten würde.

Ferner haben topisch verabreichte Antibiotika den Nachteil, daß sie die Hautflora nicht nur vom Sekundärerreger befreien, sondern auch die an sich physiologische Hautflora stark beeinträchtigen und der natürliche Heilungsprozeß auf diese Weise wieder gebremst wird.

Neben dem physikalischen Schutz verfügt die menschliche Haut über ein natürliches Abwehrsystem, das über die Produktion von antimikrobiellen Peptiden (AMPs) agiert. AMPs üben einen direkten Einfluss auf die Mikroflora der Haut aus und können den Hautzustand positiv beeinflussen. Antimikrobielle Peptide auf epithelialen Oberflächen tragen zu einer gestärkten Barrierefunktion der Haut bei und vermitteln Schutz vor Infektionen durch direkte antimikrobielle Aktivität (Radek, 2010).

Bei Säugern lassen sich die AMPs in zwei große Gruppen unterteilen: die Cathelicidine und die Defensine (Zanetti 2004, Ganz 2003). Cathelicidine sind kationische amphipatische Peptide, die in Vertebraten hoch konserviert vorliegen. Nach Prozessierung des inaktiven Precursors hCAP18 (humanes Cathelicidin antimikrobielles Peptid 18) durch Proteinasen liegt das reife Peptid LL-37 vor (Yamasaki 2006). Ähnlich wie bei den Cathelicidinen werden Defensine ebenfalls proteolytisch von einem inaktiven Proprotein zu einem aktiven Peptid prozessiert. Defensine werden aufgrund der Verknüpfung ihrer konservierten Cysteine, die intramolekulare Disulfidbrücken ausbilden, und aufgrund ihrer Sequenzhomologien in α- und β-Defensine unterteilt (Yang 2002). α-Defensine kommen vor allem in den neutrophilen Granulozyten oder Paneth Zellen des Dünndarms vor, während β-Defensine von Epithelzellen des Auges, der oralen Mukosa, des Urogenitaltraktes, des respiratorischen Systems und vor allem von Epithelzellen der Haut sekretiert werden. Für humane β-Defensine sind bisher 28 Gene in silico gefunden worden (Schutte und McCray 2002), wobei die meisten bis heute wenig untersucht wurden. Die am besten beschriebenen β-Defensine sind die humanen β-Defensine 1 - 4. Während hBD1 konstitutiv exprimiert wird, werden hBD2 und hBD3 durch Bakterien oder deren Produkte, als auch durch proinflammatorischen Zytokine induziert (Harder 2000). Die Induktion von hBD4 wird u. a. durch Hitze-inaktivierte Streptokokken (S. pneumoniae) beschrieben (Garcia 2001).

Der große Vorteil der humanen β-Defensine liegt in der antimikrobiellen Wirkungsweise und der Fähigkeit das Immunsystem zu modulieren: β-Defensine weisen eine große Bandbreite an mikrobieller Aktivität gegen eine große Anzahl von Bakterien auf und sind somit ein wichtiger Bestandteil der angeborenen Immunantwort zum Schutz des Wirtes als Reaktion auf Infektionen.

Die topische Applikation eines Wirkstoffes, der in der Lage ist, die Menge der an, auf bzw. in der Haut verfügbaren AMPs zu steigern, kann wesentlich zur Stärkung der kutanen Abwehr und zur Modulation physiologisch und kosmetisch relevanter Keime beitragen. Aufgabe war es daher, einen Wirkstoff zu finden, der die Menge oder Bioverfügbarkeit antimikrobieller Peptide erhöht.

Die Patentschrift US20080050398A1 beschreibt eine Zusammensetzung, in der hBD2 und/oder hBD3 enthalten ist und die zur Reduktion/Prävention von mikrobiellen Kontaminationen beitragen soll.

In WO0206821A2 wird beschrieben, dass bestimmte kationische Polymere die Inaktivierung von antimikrobiellen Peptiden verhindern, indem sie bakterielle Proteinasen hemmen.

In EP1974720A1 wird die Verwendung einer Komponente der Lipopolysaccharidschicht, Lipid A, des nicht-pathogenen Bakteriums *Vitreoscilla filiformis* genannt, um die Synthese von antimikrobiellen Peptiden auf der Haut anzuregen.

DE102009029110A1 offenbart eine antibakterielle und antifungale Wirkung einer Komposition bestehend aus einem *Peumus boldus* Extrakt und einem Dipeptid (Acetyl Dipeptide-3 Aminohexanoate), die zur Stimulation der hBD2 und hBD3 Synthese führt.

Aus WO0168085A1 ist bekannt, dass Isoleucin die Produktion von Defensinen in eukaryotischen Zellen anregen kann.

FR2863893 beschreibt pflanzliche Extrakte, die die Expression von hBD2 und/oder hBD3 erhöhen sollen. Gleichzeitig wird eine Methode offenbart, die zur Auswahl von geeigneten Wirkstoffen führen soll.

In WO2009037183 wird eine kosmetische und/oder pharmazeutische Zusammensetzung beschrieben, die Nukleinsäuren in Form von Oligonukleotiden enthält und die Induktion antimikrobieller Peptide in epithelialen Deckgeweben anregen soll.

FR2916634B1 offenbart die Verwendung eines Boldoextraktes (Betapur^{R}) und eines Mädesüßextraktes (Dermapur^{R}) zur Induktion von Cathelicidin, sowie alpha- und beta-Defensinen. Gleichzeitig finden Fructooligosaccharide zur Aufrechterhaltung einer gesunden mikrobiellen Hautflora in der Zusammensetzung Anwendung.

Aus FR2896691 B1 ist der Einsatz von Alkylglycosidester zum Schutz vor Mikroorganismen mittels Expressionsinduktion von endogenen AMPs wie hBD2, hBD3 und LL-37 bekannt.

Eine Aufgabe der vorliegenden Erfindung war also, den Stand der Technik in dieser Richtung zu bereichern, insbesondere, Substanzen zur Verfügung zu stellen, welche gegen grampositive und/oder gramnegative Bakterien wirksam sind, ohne dass mit der Anwendung der Substanzen eine unvertretbare Beeinträchtigung der Gesundheit des Anwenders verbunden wäre.

Es wurde überraschend gefunden, dass ε-Polylysin (Epsilon-Polylysin), welches einen Polymerisationsgrad von 25 bis 35 aufweist, zur medizinischen topischen Verwendung zur Induktion humaner β-Defensine in menschlicher Haut zur Stärkung der kutanen Abwehr diese Aufgaben löst. Gegenstände, die in der vorliegenden Beschreibung offenbart werden, jedoch nicht durch den Umfang der Ansprüche abgedeckt sind, stellen nicht Teil der gegenwärtig beanspruchten Erfindung dar.

Die JP 2003 171464 A konnte nicht den Weg zur vorleigenden Erfindung weisen.

L-Lysin ist durch folgende chemische Struktur gekennzeichnet:

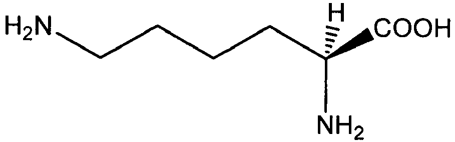

ε-Polylysin stellt ein Polymer dar, bei dem die ε-ständige Aminogruppe eines Lysinmoleküls mit der Säurefunktion eines weiteren Lysinmoleküls verknüpft ist

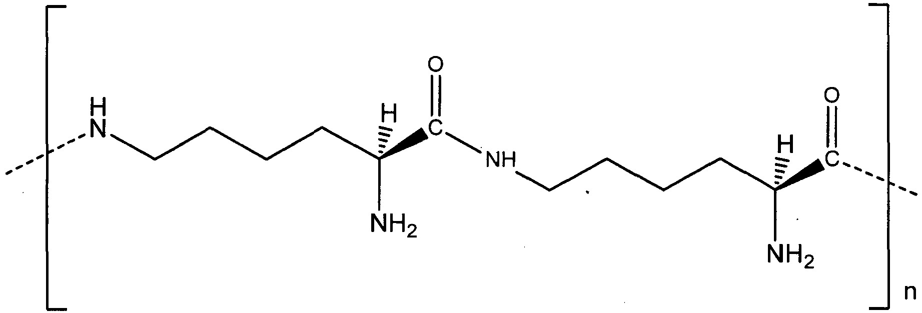

Durch den Index "n" (genauer gesagt: durch den Wert n/2) wird der Polymerisationsgrad des Polylysins definiert.

Die INCI-Bezeichnung lautet Poly[imino[(2S)-2-amino-1-oxo-1,6-hexandiyl]], es hat die CAS-Nr. 28211-04-3

Das Homopolymer ε-Polylysin wird bekannterweise als antimikrobielles Agens gegen Hefen, Pilze und gram-positive und gram-negative Bakterien beschrieben und eingesetzt. Derzeit findet es als Konservierungsmittel, vor allem in Japan, Korea und den USA, Anwendung. Die Herstellung des ε-Polylysins erfolgt über einen natürlichen fermentativen Prozess.

### Referenzen:

1. Shima, S. and Sakai H. (1977). "Polylysine produced by Streptomyces". Agricultural and Biological Chemistry 41: 1807-1809.
2. Shima, S. et al. (1984). "Antimicrobial action of ε-poly-L-lysine". Journal of Antibiotics 37 (11): 1449-1455.
3. GRAS Notice No. GRN 000135
4. Hiraki, J. et al. (2003). "Use of ADME studies to confirm the safety of ε-polylysine as a preservative in food". Regulatory Toxicology and Pharmacology 37 (2): 328-340.
5. Hiraki, J. (1995). "Basic and applied studies on ε-polylysine". Journal of Antibacterial Antifungal Agents 23: 349-354

Erfindungsgemäß vorteilhaft werden ε-Polylysine verwendet, die einen Polymerisationsgrad n/2 von 2 bis 200 aufweisen, bevorzugt 10 bis 100, besonders bevorzugt von 23 bis 35.

Erfindungsgemäß wird ε-Polylysin bevorzugt in kosmetischen oder dermatologischen Zusammensetzungen eingesetzt einem Gehalt von 0,0005 - 50,0 Gew.-%, insbesondere 0,01 - 20,0 Gew.-%, bezogen auf das Gesamtgewicht der Zusammensetzung bevorzugt sind. Vorteilhaft enthalten die Zusammensetzungen 0,02 - 10,0 Gew.-%, besonders bevorzugt 0,02 - 5,0 Gew.-% an ε-Polylysin, ganz besonders vorteilhaft 0,05 - 0,5 Gew.-%, jeweils bezogen auf das Gesamtgewicht der Zusammensetzung.

ε-Polylysin läßt sich ohne Schwierigkeiten in gängige kosmetische oder dermatologische Formulierungen einarbeiten, vorteilhaft in Pumpsprays, Aerosolsprays, Cremes, Salben, Tinkturen, Lotionen, Nagelpflegeprodukte (z.B. Nagellacke, Nagellackentferner, Nagelbalsame) und dergleichen.

Es ist auch möglich und gegebenenfalls vorteilhaft, εPL mit anderen Wirkstoffen zu kombinieren, beispielsweise mit anderen antimikrobiell, antimykotisch bzw. antiviral wirksamen Stoffen.

Ganz besonders vorteilhaft liegen die erfindungsgemäß verwendeten Einzelwirkstoffe in Form von Antischuppen-Shampoos, Haartonics und Emulsionen vor.

Es ist auch gegebenenfalls vorteilhaft, die Zusammensetzungen gemäß der Erfindung abzupuffern. Vorteilhaft ist ein pH-Bereich von 3,5 - 7,5. Besonders günstig ist es, den pH-Wert in einem Bereich von 4,0 - 6,5 zu wählen.

Die Formulierungen gemäß der Erfindung können wie üblich zusammengesetzt sein und zur Behandlung der Haut und/oder der Haare im Sinne einer dermatologischen Behandlung dienen.

Zur Anwendung werden die Formulierungen gemäß der Erfindung in der für Kosmetika und Dermatika üblichen Weise auf die Haut und/oder die Haare in ausreichender Menge aufgebracht.

Vorteilhaft sind solche kosmetische und dermatologische Zubereitungen, die in der Form eines Sonnenschutzmittels vorliegen. Vorteilhaft enthalten diese zusätzlich mindestens einen UVA-Filter und/oder mindestens einen UVB-Filter und/oder mindestens ein anorganisches Pigment.

Kosmetische Zubereitungen gemäß der Erfindung zum Schutze der Haut vor UV-Strahlen können in verschiedenen Formen vorliegen, wie sie z.B. üblicherweise für diesen Typ von Zubereitungen eingesetzt werden. So können sie z.B. eine Lösung, eine Emulsion vom Typ Wasser-in-Öl (W/O) oder vom Typ Öl-in-Wasser (O/W), oder eine multiple Emulsionen, beispielsweise vom Typ Wasser-in-Öl-in-Wasser (W/O/W), ein Gel, eine Hydrodispersion, einen festen Stift oder auch ein Aerosol darstellen.

Die Zubereitungen gemäß der Erfindung können kosmetische Hilfsstoffe enthalten, wie sie üblicherweise in solchen Zubereitungen verwendet werden, z.B. Konservierungsmittel, Bakterizide, Antioxidantien, Parfüme, Mittel zum Verhindern des Schäumens, Farbstoffe, Pigmente, die eine färbende Wirkung haben, Verdickungsmittel, oberflächenaktive Substanzen, Emulgatoren, weichmachende Substanzen, anfeuchtende und/oder feuchhaltende Substanzen, Fette, Öle, Wachse oder andere übliche Bestandteile einer kosmetischen Formulierung wie Alkohole, Polyole, Polymere, Schaumstabilisatoren, Elektrolyte, organische Lösungsmittel oder Silikonderivate.

Emulsionen sind gemäß der Erfindung vorteilhaft und enthalten z.B. die genannten Fette, Öle, Wachse und anderen Fettkörper, sowie Wasser und einen Emulgator, wie er üblicherweise für einen solchen Typ der Formulierung verwendet wird.

Gele gemäß der Erfindung enthalten üblicherweise Alkohole niedriger C-Zahl, z.B. Ethanol, Isopropanol, 1,2-Propandiol, Glycerin und Wasser bzw. ein vorstehend genanntes Öl in Gegenwart eines Verdickungsmittels, das bei ölig-alkoholischen Gelen vorzugsweise Siliciumdioxid oder ein Aluminiumsilikat, bei wäßrig-alkoholischen oder alkoholischen Gelen vorzugweise ein Polyacrylat ist.

Feste Stifte gemäß der Erfindung enthalten z.B. natürliche oder synthetische Wachse, Fettalkohole oder Fettsäureester. Bevorzugt werden Lippenpflegestifte sowie desodorierende Stifte ("Deo-Sticks").

Als Treibmittel für erfindungsgemäße, aus Aerosolbehältern versprühbare kosmetische oder dermatologische Zubereitungen sind die üblichen bekannten leichtflüchtigen, verflüssigten Treibmittel, z.B. Kohlenwasserstoffe (Propan, Butan, Isobutan) geeignet, die allein oder in Mischung miteinander eingesetzt werden können. Auch Druckluft ist vorteilhaft zu verwenden.

Vorteilhaft können die erfindungsgemäßen Zubereitungen zudem Substanzen enthalten, die UV-Strahlung im UVB-Bereich absorbieren, wobei die Gesamtmenge der Filtersubstanzen z.B. 0,1 Gew.-% bis 30 Gew.-%, vorzugsweise 0,5 bis 10 Gew.-%, insbesondere 1 bis 6 Gew.-% beträgt, bezogen auf das Gesamtgewicht der Zubereitung, um kosmetische Zubereitungen zur Verfügung zu stellen, die die Haut vor dem gesamten Bereich der ultravioletten Strahlung schützen.

Die kosmetischen Zubereitungen enthalten Wirkstoffe und Hilfsstoffe, wie sie üblicherweise für diesen Typ von Zubereitungen zur Haarpflege und Haarbehandlung verwendet werden.

Als Hilfsstoffe dienen Konservierungsmittel, oberflächenaktive Substanzen, Substanzen zum Verhindern des Schäumens, Emulgatoren, Verdickungsmittel, Fette, Öle, Wachse, organische Lösungsmittel, Bakterizide, Parfüme, Farbstoffe oder Pigmente, deren Aufgabe es ist, die Haare oder die Zubereitung selbst zu färben, Elektrolyte, Zubereitungen gegen das Fetten der Haare.

Zubereitungen, die ein Shampoonierungsmittel oder eine Wasch-, Dusch- oder Badezubereitung darstellen, enthalten vorzugsweise mindestens eine anionische, nichtionische oder amphotere oberflächenaktive Substanz oder Gemische daraus, mindestens eine dialkylsubstituierte Carbonsäure im wäßrigen Medium und Hilfsmittel, wie sie üblicherweise dafür verwendet werden.

Die folgenden Beispiele sollen die Verkörperungen der vorliegenden Erfindungen verdeutlichen. Die Angaben beziehen sich stets auf Gewichts-%, sofern nicht andere Angaben gemacht werden.

### Beispielrezeptur:

**Rezepturbeispiel : Haar-Shampoo**

| **Chemische Bezeichnung** | **Gew.-%** |
|---|---|
| Cocamidopropyl Betain | 3,00 |
| Natrium Laureth Sulfat | 8,00 |
| PEG-40 hydriertes Rizinusöl | 0,60 |
| Polyquaternium-10 | 0,20 |
| Natriumbenzoat | 0,45 |
| Natriumchlorid | 2,00 |
| Zitronensäure | 0,1 |
| Natriumsalicylat | 0,20 |
| Epsilon-Poly-L-Lysine | 0,10 |
| Parfum | 0,70 |
| Wasser | Ad. 100 |

**Beispielrezeptur: Antischuppen Shampoo**

| **Chemische Bezeichnung** | **Gew.-%** | **Gew.-%** |
|---|---|---|
| Natrium Laurylethersulfat | 9 | 10 |
| Cocamidopropyl Betain | 4 | 3 |
| Dinatrium PEG-5 Laurylcitrat Sulfosuccinat | - | 1 |
| Polyquaternium-10 | 0,3 | 0,1 |
| Guar Hydroxypropyl-Trimonium Chlorid | 0,2 | - |
| Climbazol | - | 0,5 |
| Piroctone Olamin | - | 0,25 |
| Epsilon-Poly-L-Lysine | 1 | 0,2 |
| Laureth-9 | - | 2,0 |
| Perlglanz | - | 2,5 |
| PEG-40 hydriertes Rizinusöl | 0,5 | 0,2 |
| Natriumsalicylat | 0,3 | 0,2 |
| Natriumbenzoat | 0,25 | 0,3 |
| Natriumchlorid | q.s. | q.s. |
| Zitronensäure | q.s. | q.s. |
| Parfüm | q.s. | q.s. |
| Wasser | ad 100 | ad 100 |

**Rezepturbeispiel: Haartonik**

| **Chemische Bezeichnung** | **Gew.-%** | **Gew.-%** |
|---|---|---|
| Ethanol | 30,0 | 40,0 |
| Panthenol | 0,2 | 0,1 |
| Tocopheryl Acetate | 0,2 | - |
| C12-13 Alkyl Lactate | 0,2 | 0,1 |
| Epsilon-Poly-L-Lysine | 0,25 | 0,5 |
| PEG-40 hydriertes Rizinusöl | - | 0,3 |
| Parfüm, Konservierungsmittel | q.s. | q.s. |
| Wasser | ad 100 | ad 100 |

**Beispielrezepturen: O/W - Emulsion**

| **Chemische Bezeichnung** | **Gew.-%** | **Gew.-%** |
|---|---|---|
| PEG-40 Stearat | 0,8 | 1 |
| Glyceryl Stearat | 2,5 | 3 |
| Caprylsäure/Caprinsäure Triglyceride | 2,5 | 2,5 |
| Cetylstearylalkohol | 3 | 3 |
| Cyclomethicone | 0,5 | 2 |
| Dimethicone | 1,5 | - |
| Nachtkerzenöl | 1 | - |
| Traubenkernöl | - | 1 |
| Isopropyl Stearat | 2,5 | 4 |
| Glycerin | 7 | 9 |
| Methylparaben | 0,1 | 0,1 |
| Phenoxyethanol | 0,3 | 0,4 |
| Propylparaben | 0,1 | 0,1 |
| Carbomer | 0,15 | 0,1 |
| Xanthan Gummi | 0,1 | - |
| Carrageenan | - | 0,3 |
| Trinatrium EDTA + Wasser | - | 1 |
| Epsilon-Poly-L-ysin | 1 | 0,5 |
| Glycyrrhiza inflata Extrakt | 0,1 | - |
| Natriumhydroxid | q.s. | q.s. |
| Parfüm | q.s. | q.s. |
| Wasser | ad 100 | ad 100 |
| Natrium Cetearyl Sulfat | 0,15 | 0,3 |
| Glyceryl Stearate, selbstemulgierend | 2 | 1,5 |
| C12-15 Alkyl Benzoat | 2 | 2 |
| Octyldodecanol | 1 | - |
| Caprylsäure/Caprinsäure Triglyceride | 2 | 2 |
| Cetylstearylalkohol | 2 | 1 |
| Cyclomethicone | 1 | 2 |
| Dimethicone | 0,5 | 1 |
| Glycerin | 5 | 7,5 |
| Isopropyl Palmitat | 2,5 | 2 |
| Methylisothiazolinone | 0,05 | 0,05 |
| Phenoxyethanol | 0,25 | 0,3 |
| Epsilon-Poly-L-Lysin | 0,1 | 0,05 |
| Carbomer | 0,3 | 0,2 |
| Carrageenan | 0,1 | - |
| Xanthan Gummi | - | 0,2 |
| Parfüm | q.s. | q.s. |
| Natriumhydroxid | q.s. | q.s. |
| Wasser | ad 100 | ad 100 |

**Beispielrezeptur: W/O - Emulsion**

| **Chemische Bezeichnung** | **Gew.-%** | **Gew.-%** |
|---|---|---|
| Polygylceryl-3 Diisostearate | 1,5 | 1,5 |
| PEG-40 Sorbitan Perisostearate | 2,5 | 2,5 |
| Lanolin Alcohol | 0,5 | 0,5 |
| Mineralöl | 8 | 8 |
| Cera Microcrystallina | 2,5 | 2,5 |
| Cyclomethicone | 4 | 4 |
| Isohexadecan | 2 | 2 |
| Isopropylpalmitat | 5 | 5 |
| lodopropynyl Butylcarbamate | - | 0,1 |
| Magnesiumsulfat | 0,5 | 0,5 |
| Zitronensäure | 0,1 | 0,1 |
| Natriumcitrat | 0,25 | 0,15 |
| Epsilon-Poly-L-Lysin | 0,1 | 0,25 |
| Glycerin | 5 | 7 |
| Kaliumsorbat | 0,1 | - |
| Parfüm | q.s. | q.s. |
| Benzylsalicylat | 0,1 | - |
| Wasser | Ad 100 | Ad 100 |

**Beispielrezeptur: Roll-on Deodorant**

| **Chemische Bezeichnung** | **Gew.- %** | **Gew.- %** |
|---|---|---|
| Polyethylenglykol(21)stearylether | 2,5 | 1,5 |
| Polyethylenglykol(2)stearylether | 1,5 | 2,5 |
| Polypropylenglykol(15)stearylether | 3 | 4 |
| Trinatriumsalz der Ethylendiamintetraessigsäure (20% wäßr. Lösung) | 1,5 | 1,5 |
| Parfum, Antioxidantien | q.s. | q.s. |
| Epsilon-Poly-L-Lysin | 0,1 | 0,5 |
| Wasser, ad | 100 | 100 |

**Beispielrezeptur: Anti-Pickelstift**

| | |
|---|---|
| Farbstoffe, Mischung | 20,0 |
| Cetearylbehenat | 12,0 |
| Laureth-2 Benzoat | 10,0 |
| Octyldodecanol | 10,0 |
| Bienenwachs, synthetisch | 6,50 |
| Polybuten | 4,00 |
| Adipinsäure-Hexamethylenediamin Polymer (Nylon-12) | 4,00 |
| Bis-Diglyceryl Polacyladipat-2 | 2,50 |
| Salicylsäure | 1,00 |
| Mica | 2,00 |
| Bisabolol | 0,50 |
| Epsilon-Poly-L-Lysin | 0,20 |
| Cyclopentasiloxan | ad 100 |

| | |
|---|---|
| Farbstoffe: CI 77891, CI 77499 | |

### In vitro Wirksamkeitsnachweis

In vitro kultivierte humane epidermale Keratinozyten wurden in Zellkultur Medium mit ε-Poly-L-Lysin in einem Konzentrationsbereich von 0,001-0,1% für 24 Stunden inkubiert. Anschließend wurde die Konzentration an hBD3 im Zellkulturüberstand mittels ELISA quantifiziert.

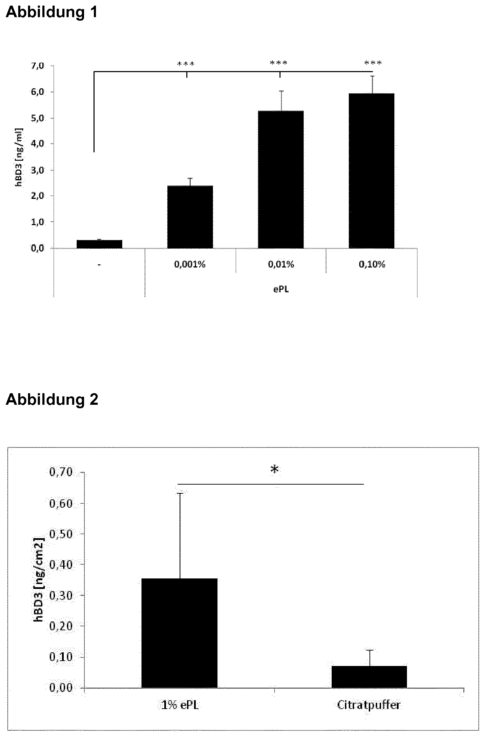

### Siehe Abbildung 1

Abb. 1: Dosisabhängige hBD3 Expression von humanen epidermalen Keratinozyten, die über einen Zeitraum von 24 Stunden mit ePL kultiviert wurden.

Abbildung 1 zeigt die dosisabhängige hBD3 Freisetzung von mit ePL inkubierten Keratinozyten. Kontrollzellen zeigten nur eine geringfüge basale hBD3 Menge. Um eine signifikante Erhöhung (p<0,001) der hBD3 Sekretion der Keratinozyten durch ePL zu bewirken, reichte eine Wirkstoffkonzentration von 0,001% aus. Die Kultivierung der Keratinozyten mit höheren Konzentrationen (0,01% und 0,1%) steigerte nochmals die hBD3 Sekretion deutlich.

### In vivo Wirksamkeitsnachweis

50 µl einer 1%igen wässrigen gepufferten Lösung (Citratpuffer pH 6)von ePL wurden auf die Innenseite des Unterarms von Probanden gegeben. Als Kontrolle diente die Applikation von Citratpuffer ohne ePL. Nach 4h Inkubation wurden die Hautareale mit 0,5 ml Pufferlösung gespült und die Konzentration hBD3 im Eluat mit Hilfe eines ELISAs quantifiziert.

### Siehe Abbildung 2

Abb. 2: Quantifizierung von hBD3 in Unterarmspülungen nach Behandlung mit 1 % ePL. Als Kontrolle wurde Citratpuffer appliziert. (n=7)

Weiterhin wurden 1%ige Lösungen von alpha-Polylysin (αPL) sowie der freien Aminosäuren Lysin und Glycin auf die gleiche Art getestet. Hierbei zeigte sich nur beim peptidisch verknüpften Lysin, nicht aber bei der freien Aminosäure eine hBD3-Steigerung, wobei alpha-und epsilon-Polylysin gleichermaßen wirksam waren.

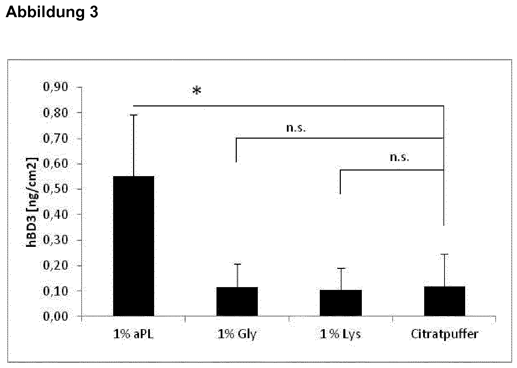

### Siehe Abbildung 3

Abb. 3: Quantifizierung von hBD3 in Flüssigkeiten von Unterarmspülungen nach Behandlung mit 1 % aPL, Glycin und Lysin. Als Kontrolle diente Citratpuffer, in dem die Wirkstoffe gelöst wurden. (n=8)

## Patentansprüche

1. ε-Polylysin (Epsilon-Polylysin), welches einen Polymerisationsgrad von 25 bis 35 aufweist, zur medizinischen topischen Verwendung zur Induktion humaner β-Defensine in menschlicher Haut zur Stärkung der kutanen Abwehr.

2. ε-Polylysin zur Verwendung nach Anspruch 1, **dadurch gekennzeichnet, dass** es in Zusammensetzungen eingesetzt wird, einem Gehalt von 0,0005 - 50,0 Gew.-%, insbesondere 0,01 - 20,0 Gew.-%, entsprechend,jeweils bezogen auf das Gesamtgewicht der Zusammensetzung.

3. ε-Polylysin zur Verwendung nach einem der vorstehenden Ansprüche, **dadurch gekennzeichnet, dass** es in kosmetischen oder dermatologischen Zusammensetzungen eingesetzt wird, einem Gehalt von 0,0001 - 5,0 Gew.-% eingesetzt, wobei insbesondere 0,001 - 2,0 Gew.-%, bezogen auf das Gesamtgewicht der Zusammensetzung bevorzugt sind.

## Claims

1. ε-Polylysine (epsilon-polylysine) having a degree of polymerization of from 25 to 35 for medical topical use for the induction of human β-defensins in human skin in order to boost the cutaneous defence.

2. ε-Polylysine for the use according to Claim 1, **characterized in that** it is used in compositions corresponding to a content of 0.0005-50.0% by weight, more particularly 0.01-20.0% by weight, based in each case on the total weight of the composition.

3. ε-Polylysine for the use according to either of the preceding claims, **characterized in that** it is used in cosmetic or dermatological compositions, used to a content of 0.0001-5.0% by weight, preference being given especially to 0.001-2.0% by weight, based on the total weight of the composition.

## Revendications

1. ε-polylysine (epsilon-polylysine) qui présente un degré de polymérisation de 25 à 35, pour l'utilisation médicale topique pour l'induction de ß-défensines humaine dans la peau humaine pour le renforcement de la défense cutanée.

2. ε-polylysine pour une utilisation selon la revendication 1, **caractérisée en ce qu'**elle est utilisée dans des compositions en une teneur de 0,0005 - 50% en poids, en particulier de 0,01 - 20,0% en poids, à chaque fois par rapport au poids total de la composition.

3. ε-polylysine pour une utilisation selon l'une quelconque des revendications précédentes, **caractérisée en ce qu'**elle est utilisée dans des compositions cosmétiques ou dermatologiques, utilisée en une teneur de 0,0001 - 5,0% en poids, en préférant en particulier de 0,001 - 2,0% en poids, par rapport au poids total de la composition.
